Europäisches Patentamt

(19) European Patent Office

Office européen des brevets

(11) Publication number:　**0 142 387**
　**A1**

## (12) EUROPEAN PATENT APPLICATION

(21) Application number: **84401710.3**

(22) Date of filing: **23.08.84**

(51) Int. Cl.⁴: **C 07 K 7/06**
　　**A 61 K 37/02, G 01 N 33/74**

(30) Priority: **26.08.83 EP 83401714**

(43) Date of publication of application:
　**22.05.85 Bulletin 85/21**

(84) Designated Contracting States:
　**AT BE CH DE FR GB IT LI NL SE**

(71) Applicant: **ANDA BIOLOGICALS**
　**37, rue de la Course**
　**F-67000 Strasbourg(FR)**

(72) Inventor: **Maes, Roland F.**
　**10 A rue du 22 novembre**
　**F-67000 Strasbourg(FR)**

(74) Representative: **Portal, Gérard et al,**
　**Cabinet Z. Weinstein 20, Avenue de Friedland**
　**F-75008 Paris(FR)**

(54) **Process for the preparation of vaccines specific for LH and HCG and process for their detection.**

㉝ The invention concerns a process of preparation of anti-HCG and anti-LH vaccines consisting in the use of oligopeptides specific of the β -subunit of LH and of HCG, located near cysteine goups, namely :

1) HCG – β : Pro – Arg – Cyst – Arg – Pro
　LH – β : Pro – Trp – Cyst – Glx – Pro

2) HCG – β : (Leu)Val– Val – Cyst– Asn – Tyr
　LH – β : Pro – Val – Cyst– Thr – Tyr

3) HCG – β : Ser – Cyst– Gln – Cyst– Ala –
　　　　　　Leu – Cyst– Arg
　LH – β : Ser – Cyst– Arg – Cyst–
　　　　　　Gly – Pro – Cyst– Arg

4) HCG – β : Thr – Cyst– Asp – Asp – Pro –
　　　　　　Arg – Phe – Gln – Asp

　LH – β : Thr – Cyst– Glx – Asx – Ser –
　　　　　　Lys – Gly – OH

characterized in that these oligopeptides are complexed upon themselves and between themselves by disulfur bridges, upon their own β -subunit of HCG and LH or upon oligopeptides containing cysteine whose aminoacid chain is common to LH and HCG, namely

HCG/LH : Gly – Cyst – Pro – Val – Cyst –
　　　　　　Ile

HCG/LH : Ile – Cyst – Ala – Gly – Tyr –
　　　　　　Cyst – Pro

HCG/LH : Pro – Gly – Cyst – Prol – Arg

HCG : Thr – Asp – Cyst – Gly – Gly

LH : Ser – Asp – Cyst – Gly – Gly

through disulfur bridges. Optionally this process comprises coupling the said oligopeptides to a high-molecular weight carrier and using them as vaccines.

According to a preferred embodiment this process comprises replacing one or several of the aminoacids present in said oligopeptides by unrelated aminoacids, or else by analogs, homologs or antimetabolite aminoacids.

This process improves the specificity of the vaccines with lowered interference level LH and HCG.

Process for the preparation of vaccines specific for
LH and HCG and process for their detection

This invention concerns a process of preparation of
vaccines for the obtention of antibodies specific for
luteinizing hormone and human chorionic gonadotropin
and a process for their detection.

The search for safe anticonceptional vaccines is
usually directed on mechanical devices used at the
time of an intercourse or else rests on synthetic
hormones destined to a daily use (the pill). A long
time sterilisation obtained by the creation of anti-
bodies that would prevent the implantation of the egg
is the subject of intense research endeavours but has
met with little success. Also diagnostic tests
detecting LH or HCG demand antibodies that are
perfectly specific for these two hormones. It has
been recognised that the fecundation of the ovule
induces the secretion of a hormone, chorionic gonadotropin, whose presence is needed for the nidation of
the egg and its perduration during the four first
months of pregnancy. This glyco-proteinic hormone is
constituted of 2 distinct subunits, named the alpha
subunit and the bêta subunit.

The alpha-subunit is identical to the alpha subunit of
the luteinizing hormone. The $\beta$-subunit is practically
identical to the $\beta$-subunit of the luteinizing
hormone. Some differences in composition of amino-acids,
all along the chain, have been discovered, as well as
a lengthening in the chain of 35 amino-acids at the
-COOH level, which do not exist with LH. A description
of the amino-acid chain of the $\beta$-subunit of HCG was
given in 1973 by Bahl, different from that given by
Morgan, also in 1973, in that the Leu in position 35 is replaced by Val. In any case, the amino-acid of position 55 in LH is Pro, different from one or the other of the two A.A. proposed for HCG (see : Immunological Fertility Regulation, 1962, Blackwell Publ., p. 39).

The existence of a sequence of 35 amino-acids, hereinafter in abbreviation A.A. peculiar to HCG fostered the hope that this particular sequence would immunise a human being without creating any interference specific for LH. French patent of 11.12.81 deposited under N° 81.21.164 (publication N° 25.16.078), describing the synthesis of part of this peptide, goes in this direction.

A publication anterior to this patent, already demonstrated that the antibodies created against this peptide did not inhibit the biologic activity of the HCG hormone. Another research endeavour, culminating by the patent of Bahl, was taken in France under N° 78.28528, with U.S. anteriority, the U.S. patent being asked on February 6th 1978 under N° 875,497. Bahl observed that 12 cysteines dotted the polypeptidic chain of the sub-unit $\beta$ of HCG. In the above-mentioned patent, a graduated or total destruction of the S-S bridges, entailing a concommitant destruction of the tertiary structure of the protein, was supposed to produce an antigen whose antibodies would react almost exclusively with HCG, and not anymore with LH. It is now however recognised that the created antibodies, without reacting anymore with LH, also do not anymore react with HCG.

This investigation strengthened the thesis that the antibodies created against HCG should, in order to be active against the biological properties of the hormone, react against some elements of the tertiary structure - tridimentional - of the protein.

A main object of the invention is to solve this technical problem in a very simple way.

According to the invention, antibodies specific for HCG and for LH are obtained by inoculation of oligopeptides specific for those hormones. The following oligopeptides, namely :

$$
\begin{array}{lll}
 & & 9 \\
HCG - \beta & : & Arg - Cyst - Arg \\
LH - \beta & : & Trp - Cyst - Glx
\end{array}
$$

$$
\begin{array}{lll}
 & & 57 \\
HCG - \beta & : & (Leu)Val - Val - Cyst - Asn \\
LH - \beta & : & Pro - Val - Cyst - Thr
\end{array}
$$

$$
\begin{array}{lll}
 & & 90 \\
HCG - \beta & : & Gln - Cyst - Ala - Leu \\
LH - \beta & : & Arg - Cyst - Gly - Pro
\end{array}
$$

$$
\begin{array}{lll}
 & & 110 \\
HCG - \beta & : & Cyst - Asp - Asp - Pro - Arg - Phe - Gln \\
LH - \beta & : & Cyst - Glx - Asx - Ser - Lys - Gly - OH
\end{array}
$$

located near a cysteine and specific for the hormone LH and HCG are linked by disulfur bridges, either among themselves or upon a $\beta$-subunit chain specific for LH and for HCG, or else upon oligopeptides containing a cysteine, that are common to the 2 hormones. Amino-acids that are identical to the two oligopeptide chains, such as Cyst in 9, 57, 90 and 110, Val in 56 and Asp in 112 may be replaced by A.A. analogs or antimetabolites.

The obtained complexes are used alone or coupled to proteins, polyssacharides, latex, bentonite, bacteria, red blood cells or other carriers and the coupled oligopeptides are then used as vaccines anti-HCG or anti-LH. The oligopeptide-sensitised solid carriers are used in diagnostic tests for the detection of LH and of HCG.

The $\beta$-subunit of HCG is constituted of a polypeptide whose amino-acids bricks are known. To this must be added the sugar residues, whose points of attachment to the polypeptidic chain are known. Twelve cysteines are present along the chain, which form S-S bridges among themselves. Together with the sugars and the hydrogen bonds, they determine the tertiary structure of the molecule. This tertiary structure is essential for the creation of specific antibodies. Indeed, the antibodies created by a $\beta$-HCG chain whose disulfur bridges have been completely broken do not react anymore with HCG and also not anymore with LH. On the other hand, a well-conducted immunisation with $\beta$-HCG, practised over several months, brings about the creation of agglutinating antibodies that react initially with LH and HCG and which become more specific for HCG in the course of time. However, the immunisation practised with the 35 amino-acids composing the -COOH terminal of $\beta$-HCG, which are particular to this hormone, brings in no case such a creation of specific agglutinating antibodies.

Unexpectedly, it appeared that the creation of specific agglutinating antibodies, on one side for HCG, on the other for LH, could be obtained in a simple way by inoculating to the animal a synthetic peptide constituted of A-Acids specific for LH or HCG, localised near the disulfur bridges.  So, following an advantageous mode of realisation of the invention, this discovery consists in a process of preparation of anti-HCG or anit-LH vaccines, consisting in the use of oligopeptides specific for the sub-unit $\beta$ of LH or HCG, adjacent to cysteine groups, such as :

1) HCG- $\beta$ : Pro - Arg - Cyst - Arg - Pro
   LH- $\beta$ : Pro - Trp - Cyst - Glx - Pro

2) HCG- $\beta$ : (Leu)Val - Val - Cyst - Asn - Tyr
   LH- $\beta$ :        Pro - Val - Cyst - Thr - Tyr

3) HCG $\beta$ : Ser - Cyst - Gln - Cyst - Ala - Leu - Cyst - Arg
   LH- $\beta$ : Ser - Cyst - Arg - Cyst - Gly - Pro - Cyst - Arg

4) HCG- $\beta$ : Thr - Cyst - Asp - Asp - Pro - Arg - Phe - Gln - Asp
   LH- $\beta$ : Thr - Cyst - Glx - Asx - Ser - Lys - Gly -OH

characterised in that these specific polypeptides are complexed upon themselves or between themselves by disulfur bridges, or else upon their own $\beta$ -HCG or $\beta$ -LH subunit or even upon oligopeptides, containing cysteine, whose amino-acids are common for LH and HCG, among which one may single out,

HCG/LH     : Gly - Cyst - Prol - Val - Cyst - Ile
HCG/LH     : Ile - Cyst - Ala - Gly - Tyr - Cyst - Pro
HCG/LH     : Pro - Gly - Cyst - Pro - Arg
HCG/LH     : Thy |‾‾‾‾‾‾
             Ser |‾‾‾‾‾‾Asp - Cyst - Gly - Gly

through disulfur bridges.

These oligopeptides may of course be used separately or together.  Preferentially, the oligopeptides bound together through disulfur bridges are used as vaccines.

Another advantageous characteristic of the invention entails the sensitisation of red blood cells, bacteria, latex or other solid phase with these peptides, and their use in diagnostic tests used for the detection of LH or HCG.

An examination of the polypeptidic chain or $\beta$-HCG and $\beta$-LH shows differences in the peptide sequence at the following levels, near cysteines :

```
                              9
HCG - β : Pro - |Arg| - Cyst - Arg
LH  - β : Pro - |Trp| - Cyst - Glx

                              57
HCG - β : |Leu(Val)| - Val - Cyst - |Asn|
LH  - β : |  Pro   | - Val - Cyst - |Thr|

          88            90              93
HCG - β : Cyst - |Gln| - Cyst - |Ala - Leu| - Cyst
LH  - β : Cyst - |Arg| - Cyst   |Gly - Pro| - Cyst

          110
HCG - β : Cyst - |Asp| - Asp - |Pro - Arg - Phe - Gln - Asp|
LH  - β : Cyst - |Glx| - Asx - |Ser - Lys - Gly - OH       |
```

The amino-acids located near the other cysteines are identical for the subunit $\beta$ of LH and HCG, namely :

```
          23              26
- Gly - Cyst - Pro - Val - Cyst - Ile

          34                    38
- Ile - Cyst - Ala - Gly - Tyr - Cyst - Pro

          72
- Gly - Cyst - Pro

          100
- Asp - Cyst - Gly
```

It is known that the primary $\beta$ amino-acid chain of HCG, obtained after reduction with mercaptoethanol or dithiothreitol or any other suitable reducing agent (named -SH6 in Bahl patent, 1978) fails to create neutralising antibodies either against HCG or against LH whereas the intact tertiary structure authorises in rate but still real cases the formation of such antibodies 100% specific for HCG, and that the inoculation of the tertiary structure of LH may induce the production of antibodies 100% specific for LH. The inventor thought that these antibodies were directed against A.A. groups located close to a disulfur bridge, such as those existing in the native molecule. Short polypeptide chains such as those here-above described are easily obtained by synthesis (see French patent 81.221.64) or may be bought. They may be complexed between themselves or else complexed upon a denatured $\beta$ -HCG chain. This denaturation is obtained by urea or guanidine acting jointly with a reducing agent such as mercaptoethanol or dithiothreitol, as is described in the Bahl patent.

The $\beta$ -subunit of HCG is isolated by dissociation of the HCG molecule in urea or guanidine and separation upon a column, according to known procedures, further described by Bahl. The splitting of the disulfur bridges of the $\beta$ -subunit of HCG is also known and is described by Bahl. It consists essentially in a treatment by a reducing agent in the presence of urea. Synthetic peptides are treated in the same way but the presence of urea or guanidine may be omitted. The -SH polypeptides that are specific of LH or HCG (peptides 7 to 10, 53 to 58, 88 to 93, and 110 to 115) are easily complexed among themselves through controlled reoxydation or else complexed upon -SH oligopeptides that are common to LH and HCG (21 to 27, 33 to 39, 71 to 73 and 99 to 101), or else they are fixed to the reduced $\beta$ -subunit of HCG.

The generation of disulfure bridges is accomplished very simply by dialysing (Spectra-Por G, Cutt off 1000) the reagents at 4°C against a phosphate isotonic buffer, or else ammonium carbonate, or other mineral salt solution or else by passing the mixture upon a suitable molecular sieve column (e.g. sephadex G 10) in order to eliminate the reducing agent and the uncomplexed surnumerous oligopeptides. A treatment with an alkylating agent such as iodoacetamine is thereafter possible but not proven really necessary.

The fixation of the oligopeptides upon a $\beta$ -subunit of HCG presents the advantage not to need anymore subsequent attachments to carrier proteins : the $\beta$ -HCG molecule is an excellent immunogen per se, that may, however, be coupled further to another carrier such as lysozyme, myristoyl dextran or tetanus toxoid. Single oligopeptides complexed among themselves through oxidation will be immunologically more active if coupled to a carrier protein. Useful products are bacteria, hemocyanin, tetanus toxoid, myristoyl dextran, lysozyme, or other.

The immunogen specific for the $\beta$ -subunit of LH and of HCG is thereafter used as a vaccine according to known procedures (Freund complete adjuvant, Freund incomplete adjuvant, MDP, etc ...).

An examination of the amino-acids composing the specific oligopeptides (8 to 10; 55 to 58; 89 to 92 and 110 to 120) and an investigation of the specificity of the antibodies produced, further shows that these A.A. are not fully specific, be it only due to the presence of the cystein bridges. In addition, the antibodies produced against the oligopeptides specific for one or the other hormone also show some interference between LH and HCG, respectively.

Accordingly, a further object of the present invention is to solve the new technical problem lying in the elimination or the sharp reduction of the interfering level shown by the anti-bodies produced against the specific oligopeptides without lowering their specificity.

Preferably, another object of the present invention is to improve simultaneously the specificity of said antibodies produced against said specific oligopeptides.

This new technical problem and said objects are achieved simultaneously for the first time according to the present invention, in an unexpected and unobvious manner.

Thus, the present invention further provides a further improvement to the above invention process which is further characterized in that it comprises replacing one or several of the amino-acids within the oligopeptides by unrelated amino-acids or else by amino-acid analogues or antimetabolites.

With this further improved process, there can be achieved a further unexpected reduction in the level of interference shown by antibodies obtained against the oligopeptides, while improving simultaneously their specificity.

By the expression "unrelated amino-acids or amino-acid analogues or antimetabolites", it is intended notably the use of amino-acids other than the amino-acids naturally present at a given position of the oligopeptide. Thus, for instance, cysteine at the 9 position of the specific oligopeptide 8 to 10 of $\beta$-HCG, which is therefore the amino-acid naturally present at the 9 position, is replaced according to the invention by another amino-acid which is unrelated, analogue, homologue or antimetabolite to it, which is therefore "unnatural" with respect to the so-called original oligopeptide, as is the case for instance of 2-mercaptohistidine or of homocysteine. In this respect, a given amino-acid can be replaced not only by a single amino-acid but also by a group of two or more amino-acids, thereby increasing the amino-acid chain.

The inventor has further unexpectedly observed that the inclusion of unnatural amino-acids within the oligopeptides that will serve as haptens and antigens in a vaccine has further the advantage to stimulate the formation of antibodies against these oligopeptides.

According to a particularly advantageous embodiment, the process according to the invention is further characterized in that it comprises the replacement, independently or in combination, of cysteine by 2-mercaptohistidine or homocysteine; the valine by $\alpha$-amino- $\beta$-chloro-butyric acid; aspartic acid by asparagine $\alpha$-amino-succinamic acid or by L-cysteic acid.

According to another feature of the invention process, which is optional, said process is further characterized in that it comprises also replacing the amino-acids that are adjacent to the above-said specific oligopeptides and which are common to LH and HCG, such as,
for example, Pro in positions 7 and 11, Tyr in
position 59 and Thr in position 109, by amino-acids
analogs, homologs or antimetabolites, and then
included in the respective oligopeptidic haptens.

It must be underlined that among the specific
oligopeptides of $\beta$ -HCG, such as :

$$\overset{9}{Arg - \underline{Cyst} - Arg}$$

$$Val - Val - \overset{57}{\underline{Cyst}} - Asn$$

$$\overset{90}{Gln - \underline{Cyst} - Ala - Leu}$$

$$\overset{110}{\underline{Cyst}} - asp - \underline{Asp} - Pro - Arg - Phe - Gln$$

One notices several amino-acids which are common to LH
and to HCG, notably Cyst at the 9, 57, 90 and 110
position, Val at the 56 position and Asp at the 112
position.

Thus, according to a further invention improvement,
these amino-acids can advantageously be replaced by
other analogues, homologues or antimetabolites amino-
acids, as well as amino-acids which are adjacent to
these specific oligopeptides and which are common to
LH and to HCG.

Thus, according to a further preferred invention
embodiment, the process comprises replacing,
independently or in combination, Pro by azetidine-2-
carboxylic acid; Tyr by 3-fluorotyrosine; Thr by
O-methyl-threonine; Ser (preferably at the 87 position) by phenylserine.

According to an alternative embodiment of the invention process, other analogue, homologue or antimetabolite amino-acids can also be used, these being easily determined by the man of the art. For instance, Tyr can be replaced by para-aminophenyl-alanine, Ser can be replaced by cyano-alanine, Arg by homo-arginine.

As previously mentioned, by introducing one or several analogue, homologue or antimetabolite amino-acids in lieu of the amino-acids naturally present in the oligopeptides, it is unexpectedly and unobviously achieved a sharp reduction of the level of interference shown by the antibodies against said specific oligopeptides. Further, simultaneously and unexpectedly, an enhancement of specificity is achieved by the anti-bodies created against them. Also, it has again been observed, in an unexpected and unobvious manner for one skilled in the art, that the replacement(s) performed according to the invention, further stimulate(s) the formation of antibodies against oligopeptides, these constituting a noticable result.

Further purposes, features and advantages of the invention will appear clearly from the reading of the following description setting forth several invention examples given merely for illustrating the invention and which cannot therefore be construed as limiting the invention scope.

EXAMPLE 1

80 mg of $\beta$ -HCG are solubilised in 10 ml of 0.5 M

Tris-HCl at pH 8.5, containing 2% EDTA and 8 M urea.

The oligopeptides Pro - Arg - Cyst - Arg - Pro -;
Val - Val - Cyst - Asn - Tyr -; Cyst - Gln - Cyst -
Ala - Leu and Cyst - Asp - Asp - Pro - Arg - (4mg each)
were added to the solution and the reagents treated
by 100 nM of DTT at RT during 30'. The mixture was
thereafter dialysed (Spectra-Por G - 1000) during 24
hours at 4°C against 8 M urea, 0.5 M Tris-HCl, pH 8.5.
The dialysis medium was thereafter changed during 48
H at 4°C, using 1% (NH4)2 CO3 at pH 7.5. The dialysis
bag was then collected, frozen and lyophilised.

Five hundred micrograms of the complexes were
inoculated to 2 rabbits in Freund's complete adjuvant
and the vaccine reinoculated 3 weeks later in Freund's
incomplete adjuvant. Two weeks later, the titer in
HCG specific agglutinating antibodies amounted in one
rabbit to 1:3.200 and in the other rabbit to 1:400.
No interference with LH could be detected through
agglutination of sheep red blood cells sensitised with
HCG, for the antiserum obtained from the second rabbit.

EXAMPLE II

Eight milligrams of the oligopeptides Pro - Trp - Cyst -
Glx - Pro and Arg - Cyst - Gly - Prol - Cyst were mixed
to the oligopeptides Gly - Cyst - Prol - Val - Cyst -
Ile (6.8 mg) and Ile - Cyst - Ala - Gly - Tyr - Cyst -
Prol (8.33 mg). Solubilisation was done in 2 ml of a
solution that was 0.5 M in Tris-HCl, pH 7.5, containing
2% EDTA, 8 M urea and 100 nM mercaptoethanol. After
30' at R.T. the reagents were dialysed (Spectra - Por
G, Cut-off 1000) against a solution 8 M in urea and
0.5 M in Tris-HCl, pH 7.5 during 24 H at 4°C, then during 38 H at 4°C against 1% (NH4)$_2$ CO3. After
dialysis, the complex was lyophilised. Recovery
amounted to 53%. Twenty milligrams were coupled to
rabbit red blood cells by a technic consisting in
activating the red blood cells during 48 H at 37°C
with glutaraldehyde, then sensitising the erythrocytes
by the peptides during 24 H at 37°C, the vaccine so
obtained was inoculated into 2 rabbits in Freund
complete adjuvant.

After 2 booster inoculations effectuated in Freund
incomplete adjuvant, blood was withdrawn. an antibody
level of 1:400 for LH was found with the first rabbit,
of 1:1.600 with the second. HCG interference amounted
respectively to 1% and 4%. The antibodies thus
obtained could be used for a diagnostic test for LH.

EXAMPLE III

Four oligopeptides specific for HCG, namely
Pro - Arg - Cyst - Arg - Pro (4.7 mg)
Leu - Val - Cyst - Asn - Tyr (4.9 mg)
Cys - Gln - Cyst - Ala - Leu - Cyst (2.2 mg)
Cyst - Asp - Asp - Pro - Arg - Phe - Gln - Asp (10.5 mg)
were solubilised together in a solution 0.5 M in Tris-
HCl, pH 7.5, 2% in EDTA and 200 nM in DTT. After 30'
at R.T., the solution was dialysed against 1% (NH4)$_2$
CO3 during 72 H at 4°C and lyophilised.

Ten mg of the product were coupled during 24 H at 37°C
and pH 8.0 to a suspension of 50% E. Coli bacteria
activated by 2.5% glutaraldehyde. After washing, a
vaccine was prepared in Freund incomplete adjuvant.
Two rabbits were inoculated with 0.5 ml of the vaccine
followed by a booster inoculation, taking place two months after the primo vaccination.

The serum of the two animals was tested 15 days later in an hemaglutination inhibition test using sheep red blood cells sensitised with HCG. The titer in agglutinating antibodies was respectively 1:200 and 1:800 , with excellent specificity for HCG.

EXAMPLE IV

Five milligrams of the oligopeptide Ser - Cyst - Gln - Cyst - Ala - Leu - Cyst, specific for HCG and 25 mg of the -COOH terminal peptide of HCG, Thr - Cyst - Asp - Asp - Pro - Arg - Phe - Gln - Asp - Ser - Ser - Ser - Lys - Ala - Pro were mixed and treated as in the preceding examples in order to create disulfur bridges.

Two mg of the product of reaction were coupled to 2 mg hemocyanine through 1% glutaraldehyde in 2 ml of a phosphate solution 0.1 M at pH 8.0. After dialysis and lyophilisation, the coupled oligopeptides were incorporated in a water in oil emulsion (Freund complete adjuvant) and inoculated in two rabbits, with a booster inoculation made 2 months later.

The agglutinating antibodies formed were specific for HCG as shown by their capacity to aggregate latex sensitised by oligopeptides specific for HCG.

EXAMPLE V

The oligopeptides :
1) Arg-mercaptohistidine-Arg-Azetidine carboxylic acid
2) Val-Amino Chloro Butyric acid-Cyst-Asn
3) Gln-mercaptohistidine-Ala-Leu

4) methyl Threonine-Cyst-Asp-Cysteic acid-Pro-Arg-Phe-
   Gln-Asp-Ser-Ser.

were complexed in the amounts 5 mg, 5 mg, 5 mg and 15 mg, respectively and lyophilised, as per example 3.

Ten mg of the product was coupled to oxidised myristoyl Dextran according to known procedures and used as a vaccine in F.C.A. Two rabbits were inoculated using 2.5 mg of peptide per animal. A booster inoculation in F.I.A. was practised 4 months later and the level of antibodies produced analysed 15 days later. Both rabbits had antibodies specific for HCG, at a level of 1:100 serum dilution in an agglutination test. No detectable interference reaction against LH could be put in evidence.

Of course, the invention cannot be restricted to the above embodiments given by way of example. In particular, the invention includes in its scope all the means constituting technical equivalents to the above-said described means as well as any combinations thereof. Notably, the replacement of the natural amino-acids by other amino-acids analogue, homologue or antimetabolite can be combined at will, i.e. even fully.

Claims

1. Process of preparation of anti-HCG and anti-LH vaccines consisting in the use of oligopeptides specific of the $\beta$-subunit of LH and of HCG, located near cysteine groups, namely :

1) HCG - $\beta$ : Pro - Arg - Cyst - Arg - Pro
   LH  - $\beta$ : Pro - Trp - Cyst - Glx - Pro

2) HCG - $\beta$ : (Leu)Val - Val - Cyst - Asn - Tyr
   LH  - $\beta$ : Pro      - Val - Cyst - Thr - Tyr

3) HCG - $\beta$ : Ser - Cyst - Gln - Cyst - Ala - Leu - Cyst - Arg
   LH  - $\beta$ : Ser - Cyst - Arg - Cyst - Gly - Pro - Cyst - Arg

4) HCG - $\beta$ : Thr - Cyst - Asp - Asp - Pro - Arg - Phe - Gln -Asp
   LH  - $\beta$ : Thr - Cyst - Glx - Asx - Ser - Lys - Gly - OH

characterized in that these oligopeptides are complexed upon themselves and between themselves by disulfur bridges, upon their own $\beta$-subunit of HCG and LH or upon oligopeptides containing cysteine whose aminoacid chain is common to LH and HCG, namely

HCG/LH : Gly - Cyst - Pro - Val - Cyst - Ile
HCG/LH : Ile - Cyst - Ala - Gly - Tyr - Cyst - Pro
HCG/LH : Pro - Gly - Cyst - Prol - Arg

HCG : Thr - Asp - Cyst - Gly - Gly
LH  : Ser - Asp - Cyst - Gly - Gly
through disulfur bridges.

2. The process of claim 1, characterized in that the oligopeptides linked through disulfur bridges are coupled to carrier proteins, polyssacharides, bacteria, red blood cells, latex, bentonite or other solid carriers, to obtain sensitized solid phases.

3.   The process of claim 1 and 2, characterized in that the oligopeptides are used separately or together.

4.   The process of claim 1, 2 or 3, characterized in that the oligopeptides complexed by disulfur bridges are used as vaccines.

5.   The process of claim 2, characterized in that said sensitized solid phases are used in diagnostic tests for the detection of LH and HCG.

6.   The process of any of claims 1 to 5, characterized in that it comprises complexing between themselves the following oligopeptides.
Pro - Arg - Cyst - Arg - Pro
Val - Val - Cyst - Asn - Tyr
Cyst - Gln - Cyst - Ala - Leu  and
Cyst - Asp - Asp - Pro - Arg.

7.   The process of any of claims 1 to 5, characterized in that it comprises complexing between themselves the following oligopeptides :
Pro - Trp - Cyst - Glx - Pro
Arg - Cyst - Gly - Pro - Cyst
Pro - Val - Cyst - Thr - Tyr  and
Thr - Cyst - Glx - Asx - Ser - Lys - OH.

8.   The process of any of claims 1 to 5 characterized in that it comprises complexing between themselves the following oligopeptides :
    Ser - Cyst - Gln - Cyst - Ala - Leu - Cyst
and Thr - Cyst - Asp - Asp - Pro - Arg - Phe - Gln -
    Asp - Ser - Ser - Ser - Lys - Ala - Pro

9. The process of any one of claims 1 to 8, characterized in that it comprises replacing one or several of the aminoacids present in said oligopeptides by unrelated aminoacids or else by analog, homolog or antimetabolite aminoacids.

10. The process of claim 9, characterized in that it comprises replacing, independently or in combination, cysteine by 2-mercaptohistidine or homo-cysteine; valine by $\alpha$ -amino- $\beta$ -chloro-butyric acid; aspartic acid by asparagine ( $\alpha$ -amino-succinamic acid) or L-cysteic acid.

11. The process of claim 9 or 10, characterized in that it comprises also replacing one or several of the amino acids which are adjacent to said specific oligopeptides and which are common to LH and to HCG, such as for instance Pro at the seventh and eleventh position, Tyr at the 59th position and Thr at the 109th position, by unrelated amino-acids or even analog, homolog or antimetabolites amino-acids, which can then be included in the respective oligopeptidic haptens.

12. The process of claim 11, characterized in that it comprises replacing, independently or in combination, Pro by azetidine-2-carboxylic acid; Tyr by 3-fluoro-tyrosine; Thr by O-methyl-threonine; Ser (preferably at the 87th position) by phenylserine.

13. The process of any one of claims 9 to 12, characterized in that it comprises replacing, independently or in combination, Tyr by para-aminophenyl-alanine; Ser by cyano-alanine; Arg by homo-arginine.

14. Process according to any one of claims 11 to 13, characterized in that it comprises complexing between
themselves the following oligopeptides :
Arg-mercaptohistidine-Arg-Azetidine carboxylic acid;
Val-Amino- Chloro Butyric acid-Cyst-Asn;
Gln-mercaptohistidine-Ala-Leu;
methyl-Threonine-Cyst-Asp-Cysteic acid and
Pro-Arg-Phe-Gln-Asp-Ser-Ser; or between themselves
with the following oligopeptides :
Azetidine-2-carboxylic acid-Arg-Cyst-Arg;
mercaptohistidine-Gln-Cyst-Ala-Leu-mercaptohistidine;
methyl Threonine-Thr-Cyst-Asp-Cysteic acid and
Pro-Arg-Phe-Gln-Asp-Ser-Ser.

15. Process according to any one of claims 11 to 14,
characterized in that it comprises replacing one or
several of the amino-acids present in said
oligopeptides by a group of two or more amino-acids,
thereby increasing the amino-acid chain.

16. Process of detection of LH and of HCG characterized in that it comprises using a carrier solid phase
consisting in red blood cells, latex, bacteria,
bentonite or other sensitized by the oligopeptides
specific for the subunit $\beta$ of LH or HCG, lying close
to the cysteine residues, as defined in any of the
claims 1, 3, 4, 6 to 15.

European Patent
Office

**EUROPEAN SEARCH REPORT**

0142387
Application number

EP 84 40 1710

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | US-A-4 392 997 (GOLDBERG) <br> * whole document * | 1 | C 07 K 7/06 <br> A 61 K 37/02 <br> G 01 N 33/74 |
| | --- | | |
| D,A | GB-A-2 013 690 (RESEARCH CORP.) <br> * whole document * | 1 | |
| | ----- | | |

TECHNICAL FIELDS
SEARCHED (Int. Cl.4)

C 07 K 7/00
A 61 K 37/00

The present search report has been drawn up for all claims

| Place of search | Date of completion of the search | Examiner |
|---|---|---|
| THE HAGUE | 05-12-1984 | RAJIC M. |